# EUROPÄISCHE PATENTANMELDUNG

(11) **EP 4 194 033 A1**
(43) Veröffentlichungstag der Anmeldung: **14.06.2023**
(21) Anmeldenummer: 22212903.3
(22) Anmeldetag: 12.12.2022
(51) Int. Cl.: A61M 5/24, A61M 5/315

(54) **VERABREICHUNGSVORRICHTUNG ZUR ABGABE EINES WIRKSTOFFS AUS EINEM MEHRKAMMERBEHÄLTER**

(30) Priorität: 13.12.2021 CH 0707072021
(71) Anmelder: Ypsomed AG, 3401 Burgdorf (CH)
(72) Erfinder: Scheurer, Simon, 3006 Bern (CH); Bosshard, Simon Martin, 3324 Hindelbank (CH); Schrul, Christian, 3400 Burgdorf (CH); Brügger, Martin, 3065 Bolligen (CH)
(74) Vertreter: Eugster, Monika Katharina

(57) **Zusammenfassung**

Die Erfindung betrifft eine Verabreichungsvorrichtung eines Wirkstoffs aus einem Mehrkammerbehälter mit wenigstens einer ersten Kammer mit einem festen Wirkstoff und einer zweiten Kammer mit einer Lösungsflüssigkeit für den Wirkstoff. Ferner umfasst die Verabreichungsvorrichtung eine Mischvorrichtung zum Abmischen des Wirkstoffs und der Lösungsflüssigkeit und zum Ausschütten des abgemischten Wirkstoffs und ein Gehäuse zur Aufnahme der Mischvorrichtung. Es sind ein erster und ein zweiter Sensor in der Verabreichungsvorrichtung vorgesehen, sodass der Abmischvorgang vollständig ausgeführt wird und somit der Wirkstoff in der gewünschten Konzentration in der Lösungsflüssigkeit vollständig gelöst ist.

## Beschreibung

Die vorliegende Erfindung betrifft eine Verabreichungsvorrichtung zur Abgabe eines Wirkstoffs aus einem Mehrkammerbehälter, insbesondere aus einem Zweikammerbehälter.

Allen erfindungsgemässen Verabreichungsvorrichtungen ist gemeinsam, dass sie zur Abgabe von einem fluiden Wirkstoff, insbesondere von einem Medikament geeignet sind.

Verschiedene Wirkstoffe, wie z.B. Wachstumshormone, können in einer in Flüssigkeit gelösten Form nicht über einen längeren Zeitraum aufbewahrt werden. Dennoch ist es zur erfolgreichen Behandlung von z.B. Wachstumsstörungen erforderlich, derartige Wirkstoffe in flüssiger Form in ein Körpergewebe eines Patienten einzubringen. Um derartige Wirkstoffe mit einer geringen Haltbarkeit in einem flüssigen Medium für eine Verabreichung mit einer Verabreichungsvorrichtung bereitstellen zu können, wurden Mehrkammerbehälter, insbesondere Zweikammerbehälter, entwickelt, in welchen ein Wirkstoff in getrockneter, bzw. lyophilisierter Form in einer ersten Kammer und eine Lösungsflüssigkeit getrennt von dem Wirkstoff in einer zweiten Kammer aufbewahrt wird. Meist sind die beiden Kammern durch bewegliche Stopfen voneinander getrennt. Die erste Kammer wird daher durch einen beispielsweise durch eine Membran verschlossenen Ausgang des Behälters, einer Behälterwand und einem ersten Stopfen gebildet. Die zweite Kammer wird anschliessend an die erste Kammer durch den ersten Stopfen, die Behälterwand und einen zweiten Stopfen gebildet. Entlang der Längsachse des Behälters, ist in der Behälterwand ein Bypass vorgesehen, der als Umlenkung um den ersten Stopfen für die Lösungsflüssigkeit dienen kann. Zum Abmischen des Wirkstoffs mit der Lösungsflüssigkeit wird ein Druck auf den zweiten Stopfen innerhalb des Behälters ausgeübt, der durch die inkompressible Lösungsflüssigkeit auf den ersten Stopfen übertragen wird. Die beiden Stopfen verschieben sich dadurch entlang der Längsachse des Behälters relativ zur Behälterwand. Sobald der erste Stopfen im Bereich des Bypasses zu liegen kommt, gelangt die Lösungsflüssigkeit durch den Bypass aus der zweiten Kammer in die erste Kammer mit dem Wirkstoff. Der zweite Stopfen kann dann so lange relativ zur Behälterwand und zum ersten Stopfen verschoben werden, bis er an dem ersten Stopfen aufliegt. Bei einem weiteren Verschieben des zweiten Stopfens wird der erste Stopfen mitverschoben und die Lösungsflüssigkeit mitsamt dem Wirkstoff kann durch die Behälteröffnung ausgeschüttet werden. Hierfür kann eine Injektionsnadel auf die Behälteröffnung aufgesetzt oder eine Injektionsnadel kann bereits fest mit der Behälteröffnung verbunden sein, wobei die Injektionsnadel eine Fluidverbindung zur ersten Behälterkammer bilden kann. Beim Abmischen des Wirkstoffes mit der Lösungsflüssigkeit ist darauf zu achten, dass der Wirkstoff keinen zu grossen Lösungsflüssigkeitsströmungen ausgesetzt wird. Eine Schaumbildung soll beim Abmischen möglichst vermieden werden. Der Wirkstoff liegt nun in gelöster Form vor und kann mit herkömmlichen Verabreichungsvorrichtungen in ein Gewebe eines Patienten injiziert werden.

Aus der WO2008/122360 A1 ist eine Injektionsvorrichtung mit einer Anzeigenvorrichtung zur Anzeige einer axialen relativen Position einer Mehrkammerkarpule zu einem Gehäuse der Injektionsvorrichtung bekannt.

Für einen Anwender dieser Verabreichungsvorrichtung ist es jedoch schwierig zu erkennen, wann der Abmischvorgang vollständig ausgeführt ist und somit der Wirkstoff in der gewünschten Konzentration in der Lösungsflüssigkeit vollständig gelöst ist.

Es ist somit eine Aufgabe der Erfindung, eine alternative Verabreichungsvorrichtung eines Wirkstoffes aus einem Mehrkammerbehälters bereitzustellen, bei welcher sichergestellt ist, dass der Wirkstoff in der gewünschten Konzentration in der Lösungsflüssigkeit vollständig aufgelöst ist.

Die Aufgabe wird mit der Verabreichungsvorrichtung gemäss Anspruch 1 gelöst. Vorteilhafte Weiterbildungen ergeben sich aus den abhängigen Ansprüchen und der Beschreibung.

Der hierin verwendete Begriff "distal" bezieht sich auf die Richtung, in die Injektionsnadel der Verabreichungsvorrichtung zeigt. Der hierin verwendete Begriff "proximal" bezieht sich auf die Richtung, die der distalen Richtung entgegengesetzt ist.

Die Erfindung geht von einer Verabreichungsvorrichtung zur Abgabe eines Wirkstoffs aus einem Mehrkammerbehälter aus. Die erfindungsgemässe Verabreichungsvorrichtung kann vorzugsweise als Autoinjektor, Injektionspen, Pumpe oder Patchinjektor ausgestaltet sein, der einen Mechanismus aufweist, der ein automatisches Abmischen und Ausschütten des abgemischten Wirkstoffs, insbesondere durch einen elektrischen Motor, welcher von einem elektrischen Energiespeicher mit Energie versorgt wird, und optional ein automatisches Einstechen und/oder Zurückziehen der Injektionsnadel bewirkt. Alternativ kann die Vorrichtung als manuelle Verabreichungsvorrichtung ausgebildet sein, d.h., dass die Kraft für die Ausschüttung des abgemischten Wirkstoffs durch Muskelkraft, wie z.B. durch die Hand des Anwenders selbst erfolgt.

Besonders bevorzugt ist bei diesen Verabreichungsvorrichtungen eine Mehrkammerkarpule, insbesondere eine Zweikammerkarpule mit einer lösbaren Nadeleinheit zur Ausschüttung des abgemischten Wirkstoffs oder eine Mehrkammerfertigspritze, insbesondere eine Zweikammerfertigspritze zur Ausschüttung des abgemischten Wirkstoffs vorgesehen. Alternative Mehrkammerbehälter, wie z.B. ein nadelloser Mehrkammerbehälter können vorgesehen sein. Der Mehrkammerbehälter weist wenigstens eine erste Kammer mit einem festen Wirkstoff und eine zweite Kammer mit einer Lösungsflüssigkeit für den Wirkstoff auf.

Die erfindungsgemässe Verabreichungsvorrichtung umfasst ferner ein Gehäuse und eine Mischvorrichtung. Die in dem Gehäuse angeordnete oder gelagerte Mischvorrichtung dient zum Abmischen des Wirkstoffs und der Lösungsflüssigkeit und dient zum Ausschütten des abgemischten Wirkstoffs. Hierzu wird die Mischvorrichtung axial relativ zu dem Gehäuse, insbesondere axial relativ zu dem Gehäuse in die distale Richtung bewegt. Die Mischvorrichtung ist relativ zu dem Gehäuse von einer ersten Position, welche den Start des Abmischens definiert, zu einer zweiten Position, welche das Ende des Abmischens definiert, zu einer dritten Position, welche das Ende der Ausschüttung definiert, axial bewegbar.

Die Mischvorrichtung kann beispielsweise durch eine Kolbenstange gebildet sein. Die Mischvorrichtung, insbesondere die Kolbenstange kann einem Vorschub eines Stopfens innerhalb des Mehrkammerbehälters in die distale Richtung dienen. Die relative axiale Bewegung der Mischvorrichtung zu dem Gehäuse kann vorzugsweise automatisch durch einen elektrischen Motor, welcher von einem elektrischen Energiespeicher mit Energie versorgt wird, erfolgen. Alternativ kann ein mechanischer Energiespeicher, wie z.B. eine vorgespannte Feder vorgesehen sein.

Die erfindungsgemässe Verabreichungsvorrichtung umfasst ferner einen ersten und einen zweiten Sensor. Der erste und der zweite Sensor sind in dem Gehäuse der Verabreichungsvorrichtung vorgesehen. Der erste Sensor kann vorzugweise als Schaltdetektor, optischer Sensor, Kraftsensor, Magnetsensor, induktiver Sensor und/oder elektrisch leidenden Sensor ausgebildet sein. Der erste Sensor ist derart ausgebildet, dass er den Mehrkammerbehälter erkennen kann. Der zweite Sensor kann vorzugweise als Beschleunigungssensor ausgebildet sein. Der zweite Sensor ist derart ausgebildet, dass er eine Bewegung, insbesondere eine Hin- und Herbewegung der Verabreichungsvorrichtung erfassen kann. Die Hin- und Herbewegung kann als eine Schüttelbewegung ausgebildet sein. Die Auswahl des ersten und des zweiten Sensors ist abhängig von dem Gegenstand, welcher erfasst oder detektiert werden soll. Zudem sind der erste und der zweite Sensor jeweils an geeigneter Messstelle in dem Gehäuse der Verabreichungsvorrichtung angeordnet.

Besonders bevorzugt kann der Mehrkammerbehälter ein Erkennungselement basierend auf einer NFC-Marke ("Near Filed Communication Tag") umfassen, welche durch den ersten Sensor erfasst oder detektiert wird. Die NFC-Marke kann vorzugsweise mindestens Informationen, Anweisungen oder Daten über den Wirkstoff, den Abmischvorgang und/oder Ausschüttvorgang speichern oder in einem Speicher enthalten. Es können auch weitere Informationen, Anweisungen oder Daten wie z. B. über den Hersteller des Wirkstoffs gespeichert sein. Somit können verschiedene Informationen, Anweisungen oder Daten von dem Mehrkammerbehälter auf die Verabreichungsvorrichtung übertragen werden. Diese Informationen, Anweisungen oder Daten können vorzugsweise auf einer Anzeige der Verabreichungsvorrichtung angezeigt werden. Dadurch kann verhindert werden, dass der Anwender einen falschen Mehrkammerbehälter in die Verabreichungsvorrichtung einfügt oder einen falschen abgemischten Wirkstoff verabreicht. Ferner kann die NFC-Marke Informationen, Anweisungen oder Daten umfassen, welche die Steuerung der Verabreichungsvorrichtung beeinflussen können, insbesondere den Abmischvorgang und/oder Ausschüttvorgang der Verabreichungsvorrichtung steuert. So können beispielsweise von der NFC-Marke Informationen, Anweisungen oder Daten über eine bestimmte Abmischzeit, über eine bestimmte Anzahl der Bewegungen, insbesondere der Hin- und Herbewegungen der Verabreichungsvorrichtung, über eine bestimmte Ausschütt- oder Dosiermenge des abgemischten Wirkstoffs oder über eine mögliche Wartezeit bis zur nächsten Verabreichung oder Injektion des abgemischten Wirkstoffs auf die Verabreichungsvorrichtung übermittelt oder übertragen werden. Die Verabreichungsvorrichtung kann vorzugsweise die übermittelten oder übertragenen Informationen, Anweisungen oder Daten automatisch ausführen. Dadurch kann erreicht werden, dass eine fehlerhafte Bedienung der Verabreichungsvorrichtung durch den Anwender verhindert wird.

Nach der Erkennung des Mehrkammerbehälters kann die Mischvorrichtung das Abmischen des Wirkstoffs mit der Lösungsflüssigkeit starten. Nach der Erkennung des Mehrkammerbehälters ist die Mischvorrichtung zum Abmischen des Wirkstoffs mit der Lösungsflüssigkeit von der ersten Position, welche den Start des Abmischens definiert, zu der zweiten Position, welche das Ende des Abmischens definiert, axial bewegbar. Der zweite Sensor, insbesondere der Beschleunigungssensor wird aktiviert, wenn die Mischvorrichtung die zweite Position, welche das Ende des Abmischens definiert, erreicht hat.

Erfindungsgemäss weist die Verabreichungsvorrichtung ein Kontrollsystem auf, mit welchem der Ablauf bei der Handhabung der Verabreichungsvorrichtung verfolgt, überwacht oder gesteuert werden kann. Das Kontrollsystem ist ausgelegt, um eine Zeitperiode zwischen dem Erreichen der zweiten Position der Mischvorrichtung, welche das Ende des Abmischens definiert, und einer Erfassung der Bewegungen, insbesondere der Hin- und Herbewegungen der Verabreichungsvorrichtung zu messen und um eine Anzahl der Bewegungen, insbesondere der Hin- und Herbewegungen der Verabreichungsvorrichtung zu messen. Das Kontrollsystem ist ferner derart konfiguriert, dass die Mischvorrichtung von der zweiten Position, welche das Ende des Abmischens definiert, zu der dritten Position, welche das Ende der Ausschüttung definiert, erst dann axial bewegbar ist, wenn die gemessene Zeitperiode und die gemessene Anzahl der Bewegungen jeweils einen bestimmten Schwellenwert überschreiten. Diese Schwellenwerte können auf der NFC-Marke des Mehrkammerbehälters gespeichert sein, wobei diese Schwellenwerte von der NFC-Marke auf die Verabreichungsvorrichtung übertragen oder übermittelt werden können. Damit kann sichergestellt werden, dass der Abmischvorgang vollständig ausgeführt wird und der Wirkstoff in der gewünschten Konzentration in der Lösungsflüssigkeit vollständig gelöst ist, bevor der abgemischte Wirkstoff verabreicht oder ausgeschüttet wird. Es wird sichergestellt, dass eine minimale Zeitperiode abgewartet und eine minimale Anzahl der Bewegungen, insbesondere der Hin- und Herbewegungen der Verabreichungsvorrichtung vor der Ausschüttung oder Abgabe des abgemischten Wirkstoffs durchgeführt werden.

In bevorzugten Ausführungsformen ist die Mischvorrichtung von der zweiten Position zu einer Zwischenposition, welche zwischen der zweiten und der dritten Position der Mischvorrichtung liegt, axial bewegbar, nachdem ein dritter Sensor die zweite Position erfasst hat. Die Zwischenposition definiert das Ende einer Entlüftung. Der dritte Sensor kann vorzugweise als Schaltdetektor, optischer Sensor, Kraftsensor, Magnetsensor, induktiver Sensor und/oder elektrisch leidenden Sensor ausgebildet sein. Die Auswahl des dritten Sensors ist abhängig von dem Gegenstand, welcher erfasst oder detektiert werden soll. Zudem ist der dritte Sensor in dem Gehäuse der Verabreichungsvorrichtung angeordnet, insbesondere an einer geeigneten Messstelle in dem Gehäuse der Verabreichungsvorrichtung angeordnet.

Vor der Ausschüttung des abgemischten Wirkstoffs mittels Verabreichungsvorrichtung in ein Gewebe eines Patienten ist es erforderlich, die Flüssigkeitsleitung zwischen der ersten Kammer, in welcher der abgemischte Wirkstoff ist, und der Spitze einer Injektionsnadel zu entlüften. Ansonsten wird zumindest teilweise anstatt der abgemischte Wirkstoff Luft verabreicht. Dieser Vorgang wird im Allgemeinen als Entlüftung bezeichnet.

Der abgemischte Wirkstoff kann erst durch die Verabreichungsvorrichtung ausgeschüttet werden, wenn die von dem Kontrollsystem gemessene Zeitperiode zwischen der zweiten Position der Mischvorrichtung, welche das Ende des Abmischens definiert, und die gemessene Anzahl der Bewegungen, insbesondere der Hin- und Herbewegungen der Verabreichungsvorrichtung jeweils einen bestimmten Schwellenwert überschreiten. Danach kann beispielsweise ein an der Verabreichungsvorrichtung vorgesehener Ausschüttknopf betätigt oder aktiviert werden. Der Ausschüttknopf ist vorher verriegelt und wird erst nach dem Überschreiten der entsprechenden Schwellenwerte betätigbar oder aktivierbar. Das Ausschütten des abgemischten Wirkstoffs kann insbesondere durch einen elektrischen Motor, welcher von einem elektrischen Energiespeicher mit Energie versorgt wird bewirkt werden.

Weiter kann die Verabreichungsvorrichtung eine axial bewegbare Nadelschutzhülse aufweisen, welche die Injektionsnadel der Nadeleinheit der Mehrkammerkarpule oder die Injektionsnadel der Mehrkammerfertigspritze mittels einer Federkraft abdeckt, wenn sich die Nadelschutzhülse in der distalen Position befindet. Ferner kann die Nadelschutzhülse ein Verriegelungselement aufweisen, welches dazu dient nach der Benutzung der Verabreichungsvorrichtung die Nadelschutzhülse in der distalen Position zu verriegeln, sodass die Verabreichungsvorrichtung nicht mehr benutzt werden kann. Die Nadelschutzhülse kann derart konfiguriert sein, dass ein automatisches Ausschütten z.B. durch einen Motor oder eine Feder der Verabreichungsvorrichtung ausgelöst wird, wenn die Nadelschutzhülse beispielsweise gegen eine Injektionsstelle gedrückt wird und somit in die proximalen Position bewegt wird, wodurch optional der Ausschüttknopf weggelassen werden kann. Nach dem Ende der Ausschüttung kann die Verabreichungsvorrichtung von der Injektionsstelle genommen werden, wobei die Nadelschutzhülse mittels der Federkraft in die distale Position bewegt wird und vorzugsweise verriegelt wird.

Die Verabreichungsvorrichtung kann eine Energiequelle, welche als Batterie ausgebildet sein kann, umfassen. Es kann eine Wegwerfbatterie oder eine wieder aufladbare Batterie oder ein Akkumulator vorgesehen sein.

In bevorzugten Ausführungsformen sind das Kontrollsystem, die Energiequelle, der erste und der zweite Sensor derart gekoppelt und konfiguriert ist, dass die Mischvorrichtung von der zweiten Position, welche das Ende des Abmischens definiert, zu der dritten Position, welche das Ende der Ausschüttung definiert, erst dann axial relativ zu dem Gehäuse bewegbar ist, wenn die gemessene Zeitperiode und die gemessene Anzahl der Bewegungen, insbesondere der Hin- und Herbewegungen jeweils einen bestimmten Schwellenwert überschreiten.

Besonders bevorzugt kann das Kontrollsystem als Prozessor oder Mikrokontroller ausgebildet sein. Der Prozessor kann eine Rechenelektronik, eine Steuerelektronik und einen Speicher aufweisen.

Das Kontrollsystem kann eine Auswertelektronik aufweisen, welche zur Identifikation von Vorgängen im oder Zuständen der Verabreichungsvorrichtung während der Bewegung, insbesondere der Hin- und Herbewegung der Verabreichungsvorrichtung und/oder Abmisch- und/oder Ausschüttvorgangs der Verabreichungsvorrichtung basierend auf Messungen des ersten und/oder zweiten Sensors konfiguriert ist. Es können beispielsweise axiale Bewegungen gefiltert und in einem Komparator oder einer Vergleichsschaltung mit einen Schwellenwert verglichen werden. Stimmt ein derart ermitteltes Muster mit einem vorbestimmten Muster über, wird die axiale Bewegung als zu dem bestimmten Ereignis gehörig identifiziert.

Besonders bevorzugt umfasst die Verabreichungsvorrichtung einen Zeitmesser. Der Zeitmesser misst die Zeitperiode zwischen der zweiten Position der Mischvorrichtung, welche das Ende des Abmischens definiert, und der Erfassung der Bewegungen, insbesondere der Hin- und Herbewegungen der Verabreichungsvorrichtung. Somit kann sichergestellt werden, dass erst nach dem Überschreiten einer bestimmten Zeitperiode der abgemischte Wirkstoff ausgeschüttet werden kann.

Das Kontrollsystem kann vorzugweise derart konfiguriert sein, um den Zeitmesser zu betreiben.

Ferner kann das Kontrollsystem der Verabreichungsvorrichtung derart konfiguriert sein, um die verstrichene Zeit seit der letzten Ausschüttung zu registrieren oder zu speichern und/oder die verstrichene Zeit nach einer festgelegten Dauer nach dem registrierten Ende der Ausschüttung zu registrieren oder zu speichern. Ferner kann auch die Erkennung des Mehrkammerbehälters und/oder die Zeit des Starts der Abmischung und/oder des Endes der Abmischung und/oder des Starts der Entlüftung und/oder des Endes der Entlüftung und/oder des Starts der Ausschüttung und/oder des Endes der Ausschüttung registriert oder gespeichert werden. Dadurch kann der Abmisch- und/oder Ausschüttvorgang der Verabreichungsvorrichtung verfolgt, überwacht oder gesteuert werden. Zudem kann dem Anwender beispielsweise Anweisungen, Informationen oder Daten auf der Anzeige der Verabreichungsvorrichtung angezeigt werden, wie die Verabreichungsvorrichtung zu bedienen ist.

In bevorzugten Ausführungsformen kann nach der Erkennung des Mehrkammerbehälters, nach einer festgelegten Dauer nach jeder registrierten Position der Mischvorrichtung und/oder nach jeder registrierten Position der Mischvorrichtung die Anzeige aktiviert werden.

Die Anzeige kann als elektrische Lichtquelle, beispielsweise als LED ausgebildet sein. Alternativ kann auch ein LC-Display oder ein OLED-Display zur Anzeige von Informationen, Anweisungen oder Daten vorgesehen sein. Es können aber auch ein anderer visueller Signalgenerator und/oder ein akustischer Signalgenerator zur Erzeugung von Geräuschen oder Melodien und/oder ein taktiler Signalgenerator zur Erzeugung von Bewegungen vorgesehen sein. Die Anzeige kann Informationen, Anweisungen oder Daten zum Zustand und zur Bedienung der Verabreichungsvorrichtung anzeigen. Es kann die Erkennung des Mehrkammerbehälters, den Start des Abmischens, das Ende des Abmischens, den Start des Entlüftens, das Ende des Entlüftens, den Start des Ausschüttens und/oder das Ende des Ausschüttens angezeigt werden. Ferner kann zusätzlich oder alternativ der Ablauf einer Haltezeit oder einer Wartezeit angezeigt werden, wobei der Anwender nach Ablauf dieser Zeit die Verabreichungsvorrichtung von der Injektionsstelle wegnehmen kann. Ferner kann dem Anwender auch die Wartezeit bis zur nächsten Verabreichung oder Injektion des abgemischten Wirkstoffs angezeigt werden.

Alternativ und/oder zusätzlich können die bereits erwähnten Informationen, Anwendungen oder Daten durch eine Echtzeit-Ereignisübertragung von Verabreichungsvorrichtung an ein externes elektronisches Gerät und/oder von dem externen elektronischen Gerät an die Verabreichungsvorrichtung erfolgen. Die bereits erwähnten Informationen, Anwendungen oder Daten können auch zusätzlich und/oder alternativ auf dem externen elektronischen Gerät angezeigt und/oder registriert oder gespeichert werden. Somit können die Informationen, Anwendungen oder Daten zwischen dem Mehrkammerbehälter, der Verabreichungsvorrichtung und/oder dem externen elektronischen Gerät austauschbar sein.

Das externe elektronische Gerät kann ein Computer, eine Cloud und/oder ein mobiles Gerät umfassend ein Smartphone oder eine Smartwatch sein.

In bevorzugten Ausführungsformen kann die Verabreichungsvorrichtung eine drahtlose Kommunikationseinheit zur Kommunikation mit dem externen elektronischen Gerät. Somit können verschiedene Informationen, Anweisungen oder Daten von der Verabreichungsvorrichtung und/oder dem Mehrkammerbehälter auf das externe elektronische Gerät übertragen werden.

Die Ausschüttung des abgemischten Wirkstoffs aus der Verabreichungsvorrichtung kann derart erfolgen, indem der Anwender die Verabreichungsvorrichtung gegen die Injektionsstelle drückt und/oder der Anwender die Verabreichungsvorrichtung mittels Pflaster auf die Injektionsstelle klebt. Entweder löst die Nadelschutzhülse die Ausschüttung des abgemischten Wirkstoffs aus oder der Anwender betätigt oder aktiviert den Auslöseknopf, um den abgemischten Wirkstoff auszuschütten.

In bevorzugten Ausführungsführungsformen kann die Verabreichungsvorrichtung eine Dosiereinrichtung, welche das Einstellen einer bestimmten Dosis des im Mehrkammerbehälter vorhandenen abgemischten Wirkstoffs ermöglicht, umfassen. Die Informationen, Anweisungen oder Daten zu der Ausschütt- oder Dosiermenge können von der NFC-Marke von dem Mehrkammerbehälter auf die Verabreichungsvorrichtung übertragen oder übermittelt werden. Alternativ kann die Verabreichungsvorrichtung keine Dosiereinrichtung aufweisen, um eine bereits fest eingestellte Dosis abzugeben. Die Verabreichungsvorrichtung kann als wiederverwendbare oder als einmal verwendbare Verabreichungsvorrichtung ausgebildet sein.

In alternativen Ausführungsformen können die Informationen, Anweisungen oder Daten betreffend einer einzustellenden oder zu verabreichender Ausschütt- oder Dosiermenge von dem externen elektronischen Gerät, insbesondere von dem Computer, der Cloud und/oder dem mobilen Gerät umfassend ein Smartphone oder eine Smartwatch auf die Verabreichungsvorrichtung übertragen oder übermittelt werden.

Zu dem kann vorgesehen sein, dass die bereits verabreichte Ausschütt- oder Dosiermenge und/oder die Ausschütt- oder Dosierzeit auf das externen elektronischen Gerät, insbesondere auf dem Computer, der Cloud und/oder dem mobilen Gerät umfassend ein Smartphone oder eine Smartwatch übertragen oder übermittelt und/oder registriert oder gespeichert werden können.

In bevorzugten Ausführungsformen sollte die Erkennung des Mehrkammerbehälters und/oder mindestens eine der Positionen der Mischvorrichtung durch den Anwender bestätigt werden, bevor die Verabreichungsvorrichtung den nächsten Vorgang des Abmischens und/oder des Entlüftens und/oder des Ausschüttens durchführen kann. Das Kontrollsystem ist ferner derart konfiguriert, dass nach der Bestätigung des Erkennens des Mehrkammerbehälters und/oder mindestens einer der Positionen der Mischvorrichtung der nächste Vorgang des Abmischens und/oder des Entlüftens und/oder des Ausschüttens, insbesondere automatisch durchgeführt werden kann. Diese Bestätigung kann durch eine Betätigung oder Aktivierung eines an der Verabreichungsvorrichtung vorgesehenen Betätigungsknopfs erfolgen. Alternative Ausgestaltungen können vorgesehen sein. Die Anzeige der Verabreichungsvorrichtung kann auch eine Touch-Funktion aufweisen, um eine Bestätigung oder Aktivierung durchzuführen.

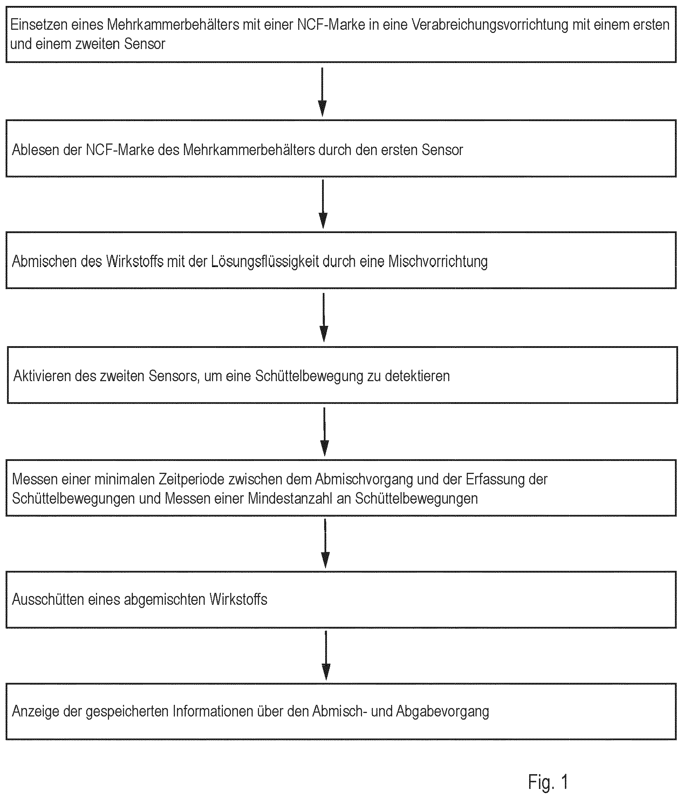

Nachfolgend werden die Erfindung und die weiteren Aspekte der Erfindung anhand einer bevorzugten Ausführung gemäss der Figur 1 beschrieben.

Der Anwender setzt einen Mehrkammerbehälter in eine Verabreichungsvorrichtung, insbesondere eine Zweikammerkarpule oder eine Zweikammerfertigspritze in ein Gehäuse der Verabreichungsvorrichtung. Der Mehrkammerbehälter weist wenigstens eine erste Kammer mit einem festen Wirkstoff und eine zweite Kammer mit einer Lösungsflüssigkeit auf. Die Verabreichungsvorrichtung erkennt den Mehrkammerbehälter mittels eines in der Verabreichungsvorrichtung vorgesehenen ersten Sensors. Der Sensor erfasst oder detektiert Informationen, Anweisungen oder Daten, welche auf einer NFC-Marke des Mehrkammerbehälters vorhanden sind. Somit können Informationen, Anweisungen oder Daten über den Wirkstoff, Abmischvorgang, wie z. B. die Abmischzeit oder die Anzahl der Schüttelbewegungen, oder der Ausschüttvorgang, wie z. B. die Dosiermenge, von dem Mehrkammerbehälter auf die Verabreichungsvorrichtung übermittelt oder übertragen werden. Der Anwender bestätigt die erfassten oder detektierten Informationen, Anweisungen oder Daten auf einer Anzeige der Verabreichungsvorrichtung. Eine in der Verabreichungsvorrichtung aufgenommene Mischvorrichtung bewegt sich axial relativ zu dem Gehäuse von einer ersten Position, welche den Start des Abmischens definiert, zu einer zweiten Position, welche das Ende des Abmischens definiert. Diese Bewegung wird vorzugsweise durch einen elektrischen Energiespeicher, wie z. B. durch einen Motor bewirkt. Die Mischvorrichtung verbleibt an der zweiten Position. Ein zweiter Sensor, insbesondere ein Beschleunigungssensor, wird aktiviert, wenn die Mischvorrichtung die zweite Position erreicht hat. Auf der Anzeige der Verabreichungsvorrichtung wird dem Anwender angezeigt, dass die Verabreichungsvorrichtung zu schütteln ist, wobei dies durch den Anwender mittels Aktivierung der Anzeige der Verabreichungsvorrichtung bestätigt wird. Der zweite Sensor, insbesondere der Beschleunigungssensor erkennt die Schüttelbewegungen der Verabreichungsvorrichtung. Nachdem eine vorbestimmte Zeitperiode zwischen der zweiten Position der Mischvorrichtung und der Erfassung der Bewegungen, insbesondere der Schüttelbewegungen der Verabreichungsvorrichtung und eine vorbestimmte Anzahl der Bewegungen, insbesondere der Schüttelbewegungen der Verabreichungsvorrichtung überschritten sind, wird dem Anwender auf der Anzeige der Verabreichungsvorrichtung angezeigt, dass Verabreichungsvorrichtung bereit ist, um den abgemischten Wirkstoff zu verabreichen. Nachdem der Anwender dies auf der Anzeige der Verabreichungsvorrichtung bestätigt hat, kann der Anwender die Verabreichungsvorrichtung auf die Injektionsstelle setzen oder legen. Nach einer Erkennung durch einen optionalen weiteren Sensor der Verabreichungsvorrichtung und/oder durch die Bestätigung auf der Anzeige, dass die Verabreichungsvorrichtung auf der Injektionsstelle gesetzt oder gelegt wurde, kann der Ausschüttvorgang des abgemischten Wirkstoffs ausgeführt werden. Der Anwender kann zur Abgabe des abgemischten Wirkstoffs einen an der Verabreichungsvorrichtung vorgesehenen Ausschüttknopf betätigen. Alternativ kann eine an der Verabreichungsvorrichtung vorgesehene Nadelschutzhülse die Ausschüttung des abgemischten Wirkstoffs bewirken. Um den abgemischten Wirkstoff aus der Verabreichungsvorrichtung abzugeben, bewegt sich die Mischvorrichtung axial relativ zu dem Gehäuse von der zweiten Position weiter zur dritten Position, welche das Ende der Ausschüttung definiert. Am Ende der Ausschüttung erscheint die Information oder die Anweisung, dass der Anwender die Verabreichungsvorrichtung von der Injektionsstelle wegnehmen kann. Dieser Vorgang kann durch den optionalen weiteren Sensor erkannt werden oder kann auf der Injektionsvorrichtung durch den Anwender bestätigt werden. Die Erkennung, Abmisch- oder Ausschüttvorgänge, Informationen, Anweisungen oder Daten mit oder ohne Zeitangabe können auf der Verabreichungsvorrichtung oder auf einem externen Speicher gespeichert werden. Somit ist jeder Bedienungsschritt und/oder jeder Verabreichungsvorgang der Verabreichungsvorrichtung durch den Anwender oder durch eine andere Person, wie beispielsweise ein Arzt verfolgbar, austauschbar oder steuerbar.

## Patentansprüche

1. Verabreichungsvorrichtung zur Abgabe eines Wirkstoffs aus einem Mehrkammerbehälter mit wenigstens einer ersten Kammer mit einem festen Wirkstoff und einer zweiten Kammer mit einer Lösungsflüssigkeit für den Wirkstoff, die eine Mischvorrichtung zum Abmischen des Wirkstoffs und der Lösungsflüssigkeit und zum Ausschütten des abgemischten Wirkstoffs und ein Gehäuse zur Aufnahme der Mischvorrichtung aufweist, wobei die Mischvorrichtung relativ zu dem Gehäuse von einer ersten Position, welche den Start des Abmischens definiert, zu einer zweiten Position, welche das Ende des Abmischens definiert, und weiter zu einer dritten Position, welche das Ende der Ausschüttung definiert, axial bewegbar ist, umfassend:
- einen in dem Gehäuse angeordneten ersten Sensor zur Erkennung des Mehrkammerbehälters,
- einen in dem Gehäuse angeordneten zweiten Sensor, insbesondere einen Beschleunigungssenor zur Erfassung einer Bewegung, insbesondere einer Hin- und Herbewegung der Verabreichungsvorrichtung,
- wobei nach Erkennung des Mehrkammerbehälters die Mischvorrichtung zum Abmischen des Wirkstoffs mit der Lösungsflüssigkeit von der ersten Position zu der zweiten Position axial bewegbar ist und der zweite Sensor, insbesondere der Beschleunigungssensor aktiviert wird, wenn die Mischvorrichtung die zweiten Position erreicht hat,
- ein Kontrollsystem, um eine Zeitperiode zwischen dem Erreichen der zweiten Position der Mischvorrichtung und einer Erfassung der Bewegungen, insbesondere der Hin- und Herbewegungen der Verabreichungsvorrichtung zu messen und um eine Anzahl der Bewegungen, insbesondere der Hin- und Herbewegungen der Verabreichungsvorrichtung zu messen, welches derart konfiguriert ist, dass die Mischvorrichtung relativ zu dem Gehäuse von der zweiten zu der dritten Position erst dann axial bewegbar ist, wenn die gemessene Zeitperiode und die gemessene Anzahl der Bewegungen, insbesondere der Hin- und Herbewegungen jeweils einen bestimmten Schwellenwert überschreiten.

2. Verabreichungsvorrichtung nach Anspruch 1, **dadurch gekennzeichnet, dass** die Mischvorrichtung relativ zu dem Gehäuse von der zweiten Position zu einer Zwischenposition, wobei die Zwischenposition zwischen der zweiten und der dritten Position der Mischvorrichtung liegt und wobei die Zwischenposition das Ende einer Entlüftung definiert, axial bewegbar ist, nachdem ein dritter Sensor die zweite Position erfasst hat.

3. Verabreichungsvorrichtung nach Anspruch 1 oder 2, **dadurch gekennzeichnet, dass** die Verabreichungsvorrichtung ferner eine Anzeige zur Anzeige von mindestens einer Position der Mischvorrichtung aufweist.

4. Verabreichungsvorrichtung nach einem der vorhergehenden Ansprüche, **dadurch gekennzeichnet, dass** der Mehrkammerbehälter eine NFC-Marke zur Erkennung des Mehrkammerbehälters umfasst.

5. Verabreichungsvorrichtung nach Anspruch 3 und 4, **dadurch gekennzeichnet, dass** die Anzeige der Verabreichungsvorrichtung Informationen, Anweisungen oder Daten von der NFC-Marke des Mehrkammerbehälters anzeigt.

6. Verabreichungsvorrichtung nach Anspruch 4, **dadurch gekennzeichnet, dass** die NFC-Marke Informationen, Anweisung oder Daten zur Steuerung der Verabreichungsvorrichtung umfasst.

7. Verabreichungsvorrichtung nach einem der vorhergehenden Ansprüche, **dadurch gekennzeichnet, dass** die Verabreichungsvorrichtung eine drahtlose Kommunikationseinheit zur Kommunikation mit einem externen elektronischen Gerät aufweist.

8. Verabreichungsvorrichtung nach einem der vorhergehenden Ansprüche, **dadurch gekennzeichnet, dass** die Verabreichungsvorrichtung ferner einen Auslöseknopf oder eine Nadelschutzhülse aufweist, um dem abgemischten Wirkstoff auszuschütten oder zu verabreichen.

9. Verabreichungsvorrichtung nach einem der vorhergehenden Ansprüche, **dadurch gekennzeichnet, dass** die relative axiale Bewegung der Mischvorrichtung zu dem Gehäuse durch einen in der Verabreichungsvorrichtung angeordneten elektrischen Motor, welcher von einem elektrischen Energiespeicher mit Energie versorgt wird, erfolgt.

10. Verabreichungsvorrichtung nach einem der vorhergehenden Ansprüche, **dadurch gekennzeichnet, dass** der Mehrkammerbehälter eine Zweikammerkarpule oder eine Zweikammerfertigspritze ist.
